Europäisches Patentamt

European Patent Office

Office européen des brevets

(11) Numéro de publication: **0 207 830**
**A1**

## (12) DEMANDE DE BREVET EUROPEEN

(21) Numéro de dépôt: **86401177.0**

(22) Date de dépôt: **03.06.86**

(51) Int. Cl.⁴: **A 61 B 10/00**
**A 61 B 17/32**

(30) Priorité: **04.06.85 FR 8508387**

(43) Date de publication de la demande:
**07.01.87 Bulletin 87/2**

(84) Etats contractants désignés:
**DE FR GB IT**

(71) Demandeur: **Schintgen, Jean-Marie**
**45 Avenue Victor Hugo**
**F-75116 Paris(FR)**

(71) Demandeur: **Zeltoun, Bruno**
**23 rue Daubenton**
**F-75005 Paris(FR)**

(72) Inventeur: **Schintgen, Jean-Marie**
**45 Avenue Victor Hugo**
**F-75116 Paris(FR)**

(72) Inventeur: **Zeltoun, Bruno**
**23 rue Daubenton**
**F-75005 Paris(FR)**

(74) Mandataire: **Dawidowicz, Armand**
**30, Boulevard du Château**
**F-92200 Neuilly(FR)**

(54) Perfectionnements aux pinces à biopsie.

(57) L'invention concerne une pince à biopsie du type comportant un câble de commande de deux cuillères ou mords pivotants, ledit câble coulissant à l'intérieur d'une gaine souple formée de fil d'acier inoxydable enroulé.

La pince à biopsie selon l'invention est caractérisée par le fait que la surface externe d'au moins une partie de la gaine est meulée.

Application aux pinces á biopsie.

## Perfectionnements aux pinces à biopsie.

L'invention concerne une pince à biopsie du type comportant un câble de commande de deux cuillères ou mords pivotants, ledit câble coulissant à l'intérieur d'une gaine souple formée de fil d'acier inoxydable enroulé.

Dans les pinces à biopsie connues, la gaine est constituée à base de fil d'acier inoxydable enroulé et, pour permettre une flexibilité accrue du côté distal, certaines pinces à biopsie connues comportent une gaine de diamètre pluf petit raccordée à la gaine principale par une pièce en acier inoxydable, qui nécessite deux soudures et provoque une gêne au coulissement de la gaine.

Ces pinces à biopsie connues présentent en outre l'inconvénient que la surface externe de la gaine, qui doit glisser à l'intérieur d'un conduit d'endoscope, est annelée et rugueuse. Cet état de surface, qui gêne le glissement de la gaine contre la paroi interne du conduit d'endoscope et provoque une usure prématurée des valves, présente en outre l'inconvénient de permettre la pénétration de débris entre les spires.

Pour pallier cet inconvénient, certaines pinces à biopsie ont une gaine recouverte extérieurement d'une pellicule, par exemple en téflon. Bien que le glissement soit amélioré, il n'est pas parfait et, au cours des déformations importantes de la gaine produites lors de l'enfoncement, la pellicule protectrice a tendance à se rompre, en rendant à nouveau la gaine rugueuse et accessible aux débris.

En outre, le glissement du câble dans la gaine est en général mauvais.

la présente invention vise à pallier les inconvénients des pinces à biopsie connues.

A cet effet, la pince à biopsie selon l'invention est caractérisée par le fait que la surface externe d'au moins une partie de la gaine est meulée. Cette surface externe lisse, outre son aspect agréable, présente l'avantage d'assurer un excellent glissement de la gaine dans le conduit d'endoscope et d'interdire le dépôt de débris entre les spires de fil d'acier inoxydable, de manière durable.

En outre, la gaine peut être meulée à un diamètre inférieur au voisinage de son extrémité distale, ce qui permet de donner une flexibilité accrue à cette extrémité, sans introduction d'une pièce de raccord gênante pour le coulissement, qui nécessite deux soudures et est soumise à des risques de rupture. Le passage au diamètre inférieur peut être progressif, ce qui permet de donner à la gaine une flexibilité variable de manière continue. Dans certains cas particuliers dans lesquels une flexibilité particulièrement élevée est nécessaire pour l'extrémité distale, on peut prévoir d'utiliser deux sections de gaîne ou plus, reliées par au moins une pièce de liaison. Le meulage de la surface extérieure est fait, avant assemblage ou, de préférence, après assemblage, de manière à meuler également les pièces de liaison.

L'opération de meulage est économique et peut être reproduite industriellement avec une très grande précision, ce qui n'est pas le cas du dépôt d'une pellicule de téflon.

En outre, dans une forme de réalisation préférée, le câble est recouvert d'une pellicule de matériau autolubrifiant, tel que du téflon, ce qui facilite considérablement son glissement et, en conséquence, le maniement de la pince à biopsie. En outre, les efforts de traction sont fortement diminués, ce qui augmente la longévité de l'ensemble de la pince à biopsie.

En outre, pour augmenter le glissement du câble de commande, il est utile de fixer ce câble, par exemple par soudure, à l'intérieur d'une gaine en fil métallique hélicoïdal, de préférence avec un enroulement inverse de celui de la gaine extérieure.

La gaine intérieure est ainsi fixée au câble, ce qui permet d'effectuer une coupe nette avec les mâchoires, même dans des positions courbes du câble, ce qui n'était pas le cas avec un câble simple.

REVENDICATIONS

1.- Pince à biopsie du type comportant un câble de commande de deux cuillères ou mords pivotants, ledit câble coulissant à l'intérieur d'une gaine souple formée de fil d'acier inoxydable enroulé, caractérisée par le fait que la surface externe de la gaine est meulée.

2.- Pince à biopsie selon la revendication 1, caractérisée par le fait que le diamètre de meulage extérieur de la gaine est réduit au voisinage de l'extrémité distale de ladite gaine.

3.- Pince à biopsie selon la revendication 2, caractérisée par le fait que le passage du diamètre extérieur de la gaine à une valeur inférieure est progressif.

4.- Pince à biopsie selon l'une des revendications 2 et 3, caractérisée par le fait que la gaine est constituée de deux sections ou plus reliées par au moins une pièce de liaison.

5.- Pince à biopsie selon la revendication 4, caractérisée par le fait que la gaine est meulée après assemblage.

6.- Pince à biopsie selon l'une des revendications 1 à 5, caractérisée par le fait que le câble est recouvert d'une pellicule de matériau autolubrifiant.

7.- Pince à biopsie selon la revendication 6, caractérisée par le fait que ledit matériau autolubrifiant est à base de téflon.

8.- Pince à biopsie selon l'une des revendications 1 à 5, caractérisée par le fait que le câble de commande est fixé à l'intérieur d'une gaine en fil métallique hélicoïdal.

9.- Pince à biopsie selon la revendication 8, caractérisée par le fait que le sens d'enroulement de ladite gaine intérieure est inverse de celui de la gaine extérieure.

## DOCUMENTS CONSIDERES COMME PERTINENTS

| Catégorie | Citation du document avec indication. en cas de besoin. des parties pertinentes | Revendication concernée | CLASSEMENT DE LA DEMANDE (Int Cl 4) |
|---|---|---|---|
| X | DE-A-2 926 339 (CHIKASHIGE et al.)<br>* Page 8, lignes 22-24; figures * | 1 | A 61 B 10/00<br>A 61 B 17/32 |
| A | | 2 | |
| A | FR-A-2 008 632 (WOLF)<br>* Page 2, lignes 7-13; figures * | 1 | |
| A | DE-A-2 657 983 (CHIKASHIGE et al.)<br>* Page 6, lignes 8-20; page 5, lignes 23-27; figures * | 1,4,8 | |
| A | US-A-3 964 468 (SCHULZ)<br>* Colonne 3, lignes 13-16; figures * | 1,6 | DOMAINES TECHNIQUES RECHERCHES (Int Cl 4) |
| A | US-A-4 235 244 (ABELE et al.)<br>* Colonne 3, lignes 12-16; figures * | 1,6,7 | A 61 B |
| A | US-A-2 739 585 (AYRE)<br>* Colonne 2, lignes 54-58; figure 4, repère 19 * | 1,8 | |
| A | EP-A-0 023 139 (OMATA et al.)<br>* Figure 3, repère 10a * | 1,8,9 | |
|  | --- -/- | | |

Le present rapport de recherche a été établi pour toutes les revendications

| Lieu de la recherche | Date d'achèvement de la recherche | Examinateur |
|---|---|---|
| LA HAYE | 12-09-1986 | GLAS J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur. mais publié à la date de dépôt ou après cette date
D : cité dans la demande
L : cité pour d'autres raisons

& : membre de la même famille. document correspondant

0207830

Office européen
des brevets

RAPPORT DE RECHERCHE EUROPEENNE

Numéro de la demande

EP 86 40 1177

Page 2

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int Cl 4) |
|---|---|---|---|
| A | US-A-3 584 518 (HURLOW)<br>* Colonne 1, lignes 53-64; colonne 2, lignes 7-10; figure 1 * | 8,9 | |
| | ----- | | |
| | | | **DOMAINES TECHNIQUES RECHERCHES (Int Cl 4)** |

Le present rapport de recherche a été établi pour toutes les revendications

| Lieu de la recherche | Date d achevement de la recherche | Examinateur |
|---|---|---|
| LA HAYE | 12-09-1986 | GLAS J. |